Europäisches Patentamt

European Patent Office

Office européen des brevets

(11) Veröffentlichungsnummer: **0 161 568**
A2

# (12) EUROPÄISCHE PATENTANMELDUNG

(21) Anmeldenummer: **85105166.4**

(22) Anmeldetag: **27.04.85**

(51) Int. Cl.⁴: **A 61 B 17/22**

---

(30) Priorität: **12.05.84 DE 3417710**

(43) Veröffentlichungstag der Anmeldung: **21.11.85**
Patentblatt 85/47

(84) Benannte Vertragsstaaten: **CH FR GB IT LI NL**

(71) Anmelder: **DORNIER SYSTEM GmbH, Postfach 1360,
D-7990 Friedrichshafen (DE)**

(72) Erfinder: **Hepp, Wolfgang, Dr., Hardtstrasse 6,
D-7997 Immenstaad (DE)**
Erfinder: **Forssmann, Bernd, Dr., Salemweg 6,
D-7990 Friedrichshafen (DE)**
Erfinder: **Brendel, Walter, Prof., Egenhofenstrasse 17a,
D-8033 Planegg (DE)**
Erfinder: **Chaussy, Christian, Prof., 2974 Tiffany Circle,
Los Angeles California 90077 (US)**

(74) Vertreter: **Landsmann, Ralf, Dipl.-Ing., Kleeweg 3,
D-7990 Friedrichshafen 1 (DE)**

---

(54) **Applikationsschablone.**

(57) Vorrichtung zur Abgrenzung des Eintrittsbereichs von Stoßwellen zu therapeutischen Zwecken in Körper (2) von Lebewesen, bestehend aus einer Schablone (6) aus stoßwellenundurchlässigem Material, zum Beispiel Schaumstoff, die direkt am Körper (2) befestigt ist, zum Beispiel durch eine Klebung oder mittels eines Korsetts, und eine Öffnung (7) für den Stoßwellendurchtritt enthält.

DORNIER SYSTEM GMBH

7990 Friedrichshafen


Reg. S 474 EU


Applikationsschablone


Die Erfindung betrifft eine Vorrichtung zur Abgrenzung des Eintrittsbereichs von Stoßwellen zu therapeutischen Zwecken in Körper von Lebewesen.

Bekannt ist eine Vorrichtung zur berührungslosen Zerkleinerung von Konkrementen in Körpern von Lebewesen mittels Stoßwellen der DORNIER SYSTEM GMBH. Die bekannte Vorrichtung umfasst eine Wanne und am Grund der Wanne einen ellipsoidförmigen Reflektor, in dessen einem Brennpunkt durch Funkenentladung Stoßwellen produziert werden können. Die Stoßwellenfronten werden vom Reflektor auf den zweiten Brennpunkt fokussiert. Der Patient ist so in der Wanne gelagert, daß das Konkrement (z.B. der Nierenstein) im zweiten Brennpunkt liegt. Die auftreffenden Stoßwellenfronten bringen Teile des Steins zum Abplatzen und zerkleinern so den Stein in abgangsfähige Bruchstücke.

/2

0161568

Bekannt ist aus der DE-OS 31 46 626 eine entsprechende Vorrichtung, bei der die Wanne zum Ankoppeln der Stoßwellen durch flüssigkeitsgefüllte Kissen ersetzt ist.

Bei der Überleitung von Stoßwellen über Körpergewebe tritt die Gefahr auf, daß stoßwellensensible Organe wie Lunge, Darm oder Knochen unkontrolliert dem Stoßwellenfeld ausgesetzt sind. Da die Lage der Organe zur Körperoberfläche von Patient zu Patient stark variiert und zusätzlich von der Stellung des Patienten abhängt, können sich nach der Ausrichtung des Stoßwellenfeldes auf ein Organ im Körper mittels Ortungssystem immer noch sensible Organe im Stoßwellenfeld befinden. Dies gilt insbesonders für Applikationsziele in der Nähe der Lunge oder des Darms, wie zum Beispiel bei der Dekalzifizierung von Mitralklappen des Herzens oder bei der Zerkleinerung von Gallengangsteinen.

Zur Anpassung eines Stoßwellenfeldes an Körper von Kindern wurde bereits vorgeschlagen (Patentanmeldung P 33 20 998) Blendenringe auf den Reflektorkörper aufzusetzen. Für die im allgemeinen komplexen anatomischen Strukturen ist diese Lösung jedoch nicht immer ausreichend.

Aufgabe der Erfindung ist es, eine Vorrichtung zum Abgrenzen des Eintrittsbereichs von Stoßwellen in den Körper von Lebewesen zu schaffen, die zuverlässig stoßwellensensible Organe schützt.

/3

Diese Aufgabe wird von Vorrichtungen mit den in den Ansprüchen genannten Merkmalen gelöst.

Die Erfindung ermöglicht eine individuelle Anpassung des von Stoßwellen durchlaufenen Bereiches an den jeweiligen Körper. Da die Schablonen direkt am Körper befestigt sind, bleiben die empfindlichen Bereiche auch bei einer Fehlpositionierung der Stoßwellenquelle ausreichend abgeschattet.

Die Schablone kann so auf die Haut des Patienten aufgeklebt oder auf andere Weise, zum Beispiel mit einem Korsett, befestigt werden, daß die Durchtrittsöffnung gerade das stoßwellenunbedenkliche Areal freiläßt. Das Stoßwellenfenster wird vor der Applikation in der Vorbereitungsphase durch bekannte medizinische Diagnosetechniken wie Perkussion, Auskultation, Ultraschalluntersuchungen, Röntgentechniken u.a. bestimmt und gegebenenfalls auf der Körperoberfläche des Patienten markiert.

In einer vorteilhaften Ausführung kann die Schablone für bestimmte Phasen periodischer Körperfunktionen, wie zum Beispiel Expiration/Inspiration, Systole/Diastole, speziell gewählt werden, je nachdem, welche der genannten Phasen für eine Applikation der (dann getriggert ausgelösten) Stoßwellen Vorteile verspricht.

Das Eintrittsfenster wird - um die Verschiebung der Organe unter Schwerkrafteinfluß zu berücksichtigen - in derselben Stellung des Patienten festgestellt, in der der Patient anschliessend mit Stoßwellen behandelt wird.

Bei der Auswahl oder beim Zuschnitt der Schablonen sind die Ausbreitungseigenschaften des Stoßwellenfeldes - parallel, divergent oder konvergent - zu berücksichtigen.

Stoßwellen werden von lufthaltigem Material stark absorbiert bzw. reflektiert. Es ist deshalb vorteilhaft, lufthaltigen Schaumstoff zu verwenden, der durch eine selbstklebende Schicht (ähnlich den bekannten Klebeverbänden) unverschieblich mit der Haut verbunden wird. Wenn die Applikation von Stoßwellen im Wasserbad stattfindet, ist eine wasserfeste Klebung und geschlossenporiger Schaum vorzuziehen. Da die Stoßwellen über eine Wasserschicht, Gelschicht oder eine stoßwellendurchlässige Membran, die über eine Fett- oder Ölschicht auf die Haut kontaktiert wird, in den Körper eingeleitet werden, ist die Schablone und die Klebung resistent gegen diese Stoffe ausgelegt.

Durch eine erfindungsgemäße Schablone wird, unabhängig von Justiervorgängen, Justierfehlern oder unvermeidlichen Bewegungen des Patienten die unbemerkte Belastung sensibler Organe vermieden.

Die Eintrittsschablone kann auf bestimmte Behandlung oder bestimmte Patiententypen standardisiert sein oder individuell für jeden Patienten aus entsprechend vorbereiteten und eventuell mit einer Klebeschicht versehenen Kunststoffbahnen zugeschnitten sein. Vorteilhaft ist die Verwendung von leicht zuschneidbarem Material.

Um eine Behinderung der Ortung zu vermeiden kann entweder röntgendurchlässiges oder ultraschalldurchlässiges Material verwendet werden.

Weitere Vorteile, Merkmale und Ausgestaltungen der Erfindung werden anhand von drei Figuren näher erläutert.

Es zeigen:

Figuren 1 und 2 die Wirkung erfindungsgemäßer Schablonen an einem Patientenkörper,

Figur 3 zwei Ausführungsformen von erfindungsgemäßen Schablonen.

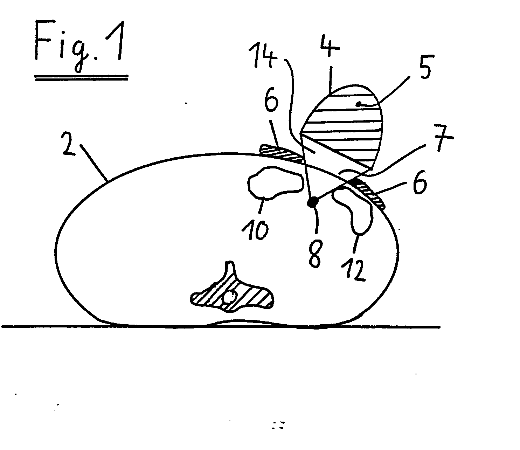

Figur 1 zeigt schematisch einen Querschnitt durch einen Patientenkörper 2, einen Reflektor 4 zum Fokussieren von Stoßwellen, die von der Stoßwellenquelle 5 im ersten Brennpunkt des Reflektors 4 erzeugt werden, und eine erfindungsgemäße Schablone 6 mit Eintrittsfenster 7. Im Körper 2 befindet sich im zweiten Brennpunkt des Reflektors 4 ein zu zerkleinerndes Konkrement 8, hier zum Beispiel ein Gallenstein . In der Nähe des Konkrements 8 befinden sich zwei stoßwellenempfindliche Organe 10, 12.

Werden nun im ersten Brennpunkt des Reflektors 4 Stoßwellen erzeugt und auf den zweiten Brennpunkt fokussiert, so laufen diese im Kegel 14 auf das Konkrement 8 zu. Die Schablone 6 schützt die empfindlichen Organe 10, 12 vor Stoßwellen, auch wenn zum Beispiel der Reflektor 4 zu nahe an den Körper 2 herangerückt sein sollte.

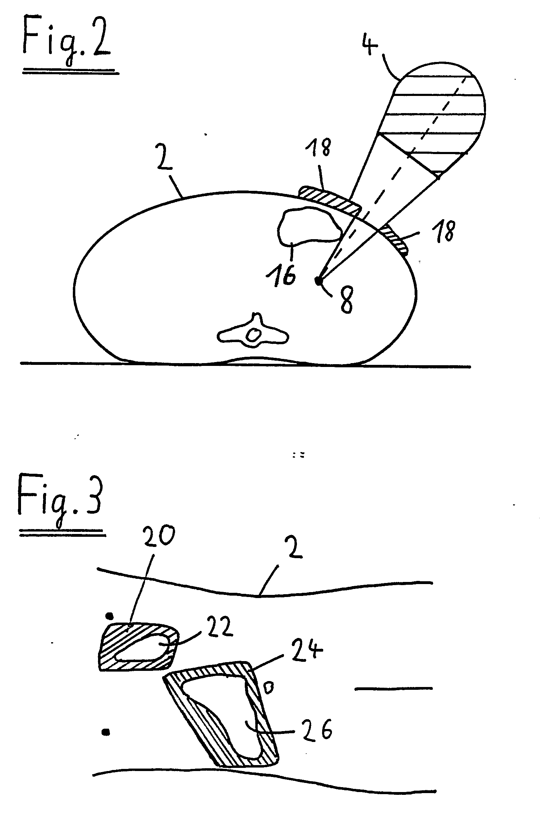

Figur 2 zeigt schematisch einen der Figur 1 entsprechenden Querschnitt eines menschlichen Körpers. In dieser Figur liegt das Konkrement 8 jedoch tiefer im Körper 2 und ein empfindliches Organ 16 im Eintrittskegel. Die Schablone 18 ist so ausgeschnitten, daß ein großer Teil des Stoßwellenfeldes ausgeblendet wird.

Figur 3 zeigt in Draufsicht zwei Ausführungsformen erfindungsgemäßer Schablonen auf einem menschlichen Körper 2, wobei die Schablone 20 ein birnenförmiges Eintrittsfenster 22 zur Beschallung des Herzens (Dekalzifizierung der Mitralklappe) enthält. Die Schablone 20 schützt Lunge und Magen. Die zweite gezeigte Schablone 24 enthält ein in etwa dreieckiges Eintrittsfenster 26 für die Gallensteinzerkleinerung, das oben den Brustkorb und die Lunge und unten das Colon schützt. Die äußere Kontur der Schablone richtet sich nach

0161568

der Größe und nach dem Abstand des Stoßwellenreflektors oder eines verwendeten Stoßwellenübertragers.

DORNIER SYSTEM GMBH

7990 Friedrichshafen

Reg. S 474 EU

P a t e n t a n s p r ü c h e :

1. Vorrichtung zur Abgrenzung des Eintrittsbereichs von Stoßwellen zu therapeutischen Zwecken in Körper von Lebewesen, d a d u r c h  g e k e n n z e i c h - n e t , daß die Vorrichtung aus einer Schablone (6) aus stoßwellenundurchlässigem Material besteht, die direkt auf dem Körper (2) des Lebewesens befestigt ist und die eine Öffnung (7) für den Stoßwellendurchtritt enthält.

2. Vorrichtung nach Anspruch 1, dadurch gekennzeichnet, daß sie aus lufthaltigem Schaumstoff mit offenen oder geschlossenen Poren besteht.

3. Vorrichtung nach Anspruch 1 oder 2, dadurch gekennzeichnet, daß sie aus röntgendurchlässigem oder aus ultraschalldurchlässigem Material besteht.

4. Vorrichtung nach einem der vorhergehenden Ansprüche, dadurch gekennzeichnet, daß sie Vorrichtungen zum Festkleben auf den Körper (2), vorzugsweise eine selbstklebende Schicht, umfasst.

5. Vorrichtung nach einem der vorhergehenden Ansprüche, dadurch gekennzeichnet, daß die Form der Öffnung (7) der Lage des Körpers (2), zum Beispiel am Rücken liegend, und dem Zustand der Organe (10, 12), insbesondere einer Phase einer Körperfunktion,wie zum Beispiel ausgeatmet, angepasst ist.

6. Vorrichtung nach einem der vorhergehenden Ansprüche, dadurch gekennzeichnet, daß die Öffnung (7) der Schablone (6) auf bestimmte Therapien wie Nierensteinzerkleinerung oder Gallensteinzerkleinerung und/oder Personentypen wie Erwachsene oder Kinder standardisiert ist.

7. Vorrichtung nach einem der vorhergehenden Ansprüche, dadurch gekennzeichnet, daß die Schablone (6) aus einem leicht auf individuelle Patientenverhältnisse zuschneidbarem Material besteht.

8. Vorrichtung nach einem der vorhergehenden Ansprüche, dadurch gekennzeichnet, daß sie wie ein den Körper umfassendes Korsett ausgebildet ist.

Fig.1

## Fig.2

## Fig.3